# EUROPÄISCHE PATENTSCHRIFT

(11) **EP 1 368 065 B1**
(45) Veröffentlichungstag und Bekanntmachung des Hinweises auf die Patenterteilung: **27.06.2007**
(21) Anmeldenummer: 02726052.0
(22) Anmeldetag: 04.03.2002
(51) Int. Cl.: A61K 49/18

(54) **PARAMAGNETISCHE NANOPARTIKEL**
PARAMAGNETIC NANOPARTICLE
NANOPARTICULES PARAMAGNETIQUES

(30) Priorität: 08.03.2001 DE 10111321
(43) Veröffentlichungstag der Anmeldung: 10.12.2003
(73) Patentinhaber: Centrum für Angewandte Nanotechnologie (CAN) GmbH, 20146 Hamburg (DE)
(72) Erfinder: HAASE, Markus, 22457 Hamburg (DE); HAUBOLD, Stephan, 22765 Hamburg (DE); BOBBERT, Cornelius, 85 456 Wartenberg (DE); STOECKELHUBER, Beate, 23562 Lübeck (DE)
(74) Vertreter: HOFFMANN EITLE
(86) Internationale Anmeldenummer: PCT/DE2002/000772
(87) Internationale Veröffentlichungsnummer: WO 2002/072154

(56) Entgegenhaltungen:
- US-A- 5 496 536
- US-A- 6 048 515
- MOREL S ET AL: "NMR relaxometric investigations of solid lipid nanoparticles (SLN) containing gadolinium(III) complexes" EUROPEAN JOURNAL OF PHARMACEUTICS AND BIOPHARMACEUTICS, ELSEVIER SCIENCE PUBLISHERS B.V., AMSTERDAM, NL, Bd. 45, Nr. 2, März 1998 (1998-03), Seiten 157-163, XP004256965 ISSN: 0939-6411
- BULTE J W M ET AL: "MAGNETIC NANOPARTICLES AS CONTRAST AGENTS FOR MR IMAGING" SCIENTIFIC AND CLINICAL APPLICATIONS OF MAGNETIC CARRIERS, XX, XX, 1997, Seiten 527-543, XP000858781
- TOKUMITSU H ET AL: "GADOLINIUM MEUTRON-CAPTURE THERAPY USING NOVEL GADOPENTETIC ACID-CHITOSAN COMPLEX NANOPARTICLES: IN VIVO GROWTH SUPPRESSION OF EXPERIMENTAL MELANOMA SOLID TUMOR" CANCER LETTERS, NEW YORK, NY, US, Bd. 150, Nr. 2, 2000, Seiten 177-182, XP000951446 ISSN: 0304-3835
- DATABASE MEDLINE [Online] Dezember 2000 (2000-12) YU X ET AL: "High-resolution MRI characterization of human thrombus using a novel fibrin-targeted paramagnetic nanoparticle contrast agent." Database accession no. NLM11108623 XP002206266 & MAGNETIC RESONANCE IN MEDICINE: OFFICIAL JOURNAL OF THE SOCIETY OF MAGNETIC RESONANCE IN MEDICINE / SOCIETY OF MAGNETIC RESONANCE IN MEDICINE. UNITED STATES DEC 2000, Bd. 44, Nr. 6, Dezember 2000 (2000-12), Seiten 867-872, ISSN: 0740-3194

## Beschreibung

### STAND DER TECHNIK

Die vorliegende Erfindung betrifft allgemein die verbesserte Herstellung von Nanopartikeln und insbesondere die Verwendung von paramagnetischen Nanopartikeln als Kontrastverstärker für NMR-basierte Untersuchungsmethoden.

Nanopartikel können durch Herstellungsverfahren des Standes der Technik noch nicht effizient in eng definierten Grössenbereichen geringer Grösse, etwa wenige Nanometer, z.B. 4 nm groß, hergestellt werden. Dies wäre jedoch für viel technische Anwendungen wünschenswert. Diese allgemeine Aufgabe wird von der vorliegenden Erfindung gelöst.

Obwohl die vorliegende Erfindung einen großen Schutzumfang sowohl hinsichtlich der Breite der beanspruchten Stoffe als auch hinsichtlich der vielen denkbaren Anwendungen aufweist, wird sie im folgenden anhand eines speziellen Standes der Technik von diesem abgesetzt. Dieser Stand der Technik wird vom Gebiet der Kern (-magnetischen) Resonanz, der sogenannten NMR (Nuclear Magnetic Resonance) gebildet.

Dieser Bereich der Technik findet als Methode Einsatz vor allem in der diagnostischen Medizin, aber etwa auch in der Materialforschung und -prüfung.

Im Rahmen seiner praktischen Anwendung zeichnet sich die NMR durch ihre Eigenschaft der nicht-invasiven Untersuchung aus. Das Verfahren basiert auf der Ermittlung der von Gewebe zu Gewebe unterschiedlichen Verteilung von Wasserstoffatomen und kommt in der Medizin als MRT (Magnetresonanz-Tomographie) zum Einsatz. Für eine kurze thematische Einführung wird verwiesen auf: "Schild Prof.Dr. Hans H.:MRI made easy, Schering Aktiengesellschaft, 1990. ISBN 3-921817-41-2" Wesentlich bei dieser Technik ist das Element Wasserstoff. Es besitzt ein Proton (Kernladungszahl Z=1) und verfügt wie alle Elemente mit ungerader Kernladungszahl über einen als Kernspin bekannten Impuls in Form einer Eigendrehung des einzelnen Atoms. Diese Drehung erzeugt ein magnetisches Moment, das das betreffende Atom zum magnetischen Dipol macht. In einem Volumen von Wasserstoffatomen sind die magnetischen Momente jedoch regellos ausgerichtet.

Legt man jedoch künstlich ein äußeres, statisches Magnetfeld an, so richten sich die Atomkerne an den Magnetlinien aus. Sie lagen dann parallel oder antiparallel zur Achse des äußeren Magnetfeldes. Mit der sog. Larmorfrequenz kreisen die Atome um die Magnetfeldlinien des hauptfeldes, die meisten Kerne richten sich dabei parallel (energieärmerer Zustand) zum äußeren Magnetfeld aus, eine geringere Anzahl antiparallel. Somit existiert ein magnetisches Summationsmoment in z-Richtung (Richtung der Magnetfeldlinien des äußeren Feldes), und die in der xy-Ebene liegenden Anteile heben sich bei vektorieller Addition auf.

Legt man nun ein elektromagnetisches Wechselfeld (HF-Radiowelle) exakt in der Larmorfrequenz an, so kommt es aufgrund von Energieübertragung (Resonanz) zu einer Auslenkung des magnetischen Summationsmoments aus der z-Richtung, wobei die Dauer des Impulses des angelegten Wechselfeldes sowie dessen Amplitude den Winkel bestimmen, um den der Summationsvektor ausgelenkt wird.

Bei Aussetzen des Wechselfeldimpulses, führt die "Zurücklenkung" des Summationsvektors senkrecht zur xy-Ebene, also wieder parallel zur z-Richtung, zu einem Wechselstrom, der mithilfe einer Spule gemessen werden kann.

Regelmäßig von Interesse sind hierbei zwei Parameter, jeweils ausgedrückt als Zeit: So beschreibt die sog. Längs- oder Spin-Gitter-Relaxationszeit T1 die Zeitkonstante der Rückkehr der z-Komponente des Summenvektors in seine Ausgangslage. Die sog. Quer- oder Spin-Spin-Relaxationszeit T2 beschreibt feldinhomogenitätsbedingte und relaxationsbedingte Dephasierungen.

Die Relaxationszeit ist von der angelegten Feldstärke und von der Art des Gewebes abhängig. Anhand der Relaxationszeit kann man Unterschiede in der Art des Gewebes feststellen. Eine ortsauflösende Messung wird dadurch erreicht, dass man kein homogenes Magnetfeld anlegt, sondern ein Gradientenfeld. Man erreicht durch dieses Vorgehen, dass die Larmorfrequenz, die proportional zum angelegten Feld ist, in jeder (dünnen) Schicht des zu untersuchenden Gewebes unterschiedlich groß ist und ein NMR-Signal deshalb eindeutig einer Gewebsschicht zugeordnet werden kann.

Die Anwendung der NMR-Technik hat sich im Rahmen der Medizin neben dem bekannten Einsatz als MRT auf folgende Einsätze ausgeweitet:

Magnetresonanzspektroskopie (MRS): Hierbei handelt es sich um eine Untersuchung, die auch biochemische Informationen liefert. So sind bei dieser Methode definierte Metabolite signalgebend. Ihre Konzentration kann wahlweise graphisch als Spektrum oder helligkeitskodiert mit morphologischen MRT-Bildern überlagert werden (s. Thurn und Bücheler, Einführung in die radiologische Diagnostik, Thieme-Verlag, 1998).

Magnetresonanzangiographie (MRA): Veränderungen des MRT-Signals durch den Blutstrom aufgrund bewegter Spins führen zu einer zusätzlichen Information, die hier Grundlage für die Gefäßdarstellung bildet.

Kardiomagnetresonanztomographie: Die Diagnose von Herzerkrankungen basiert hier nicht ausschließlich auf morphologischer Bildinformation, sondern ist mit funktionellen Analysen gekoppelt. Diese Koppelung resultiert aus einer EKG-getriggerten MRT-Untersuchung des Herzens, eine spätere Rekonstruktion (etwa in Form einer 3dimensionalen Ansicht) lässt funktionelle Schwächen des Herzens erkennen.

MR-Funtional-Imaging: Aktivierte Hirnareale lassen aufgrund eines erhöhten Blutflusses und eines höheren Sauerstoffverbrauchs eine erhöhte T2-Zeit messen und werden in T2-gewichteten Sequenzen damit geringfügig signalintensiver. Ein Subtraktionsbild der Zustände mit und ohne Aktivität erlaubt aktivierte Areale zu erkennen.

Eine MRT-Untersuchung besteht in der Regel aus mindestens einer T1-gewichteten sowie einer T2-gewichteten Serie. Es hat sich bei vielen Fragestellungen jedoch etabliert, an diese beiden Untersuchungen eine Tl-Gewichtung mit Kontrastmittelgabe anzuschließen.
In etwa einem Drittel der MR-Untersuchungen werden Kontrastmittel eingesetzt. Sie verkürzen die T1 und T2- Relaxationszeiten in Geweben mit dem Zweck, den Gewebekontrast zu erhöhen und somit sowohl Anatomie und physiologische Abläufe besser beurteilen zu können, als auch pathologische Befunde klarer darstellen zu können.

Der Mechanismus der Kontrastanhebung im Bereich der NMR mithilfe eines Kontrastmittels basiert auf dem Vorhandensein eines ungepaarten Elektrons. Dieses Elektron hat ein magnetischen Moment, das etwa 1000 mal stärker ist als das eines Protons. Dieses Moment führt zu einer rascheren Veränderung des lokalen Magnetfeldes. Die Dipole von ungepaarten Elektronen haben eine erheblich stärkere magnetische Suszeptibilität, wenn sie in dicht gepackten kristallinen Strukturen angeordnet sind. Diese Substanzen werden als superparamagnetisch bezeichnet, mit einer 100- bis 1000-fach höheren magnetischen Suszeptibilität als paramagnetische Substanzen. Sie haben einen deutlich größeren Effekt auf den Bildkontrast (T2-Verkürzung) als paramagnetische Substanzen.

Auch zur Erhöhung der Signalintensität lassen sich paramagnetische Substanzen einsetzen. Sie sind bereits in geringer Konzentration in der Lage, die Signalintensität anzuheben. In höherer Konzentration erreicht die Signalstärke ein Plateau, um bei weiterer Konzentrationserhöhung wieder abzufallen. Dies gilt für T1- wie T2-gewichtete Aufnahmen annähernd gleich, wobei das beschriebene Plateau mit anschließendem Abfall der Signalintensität in der T1-Aufnahme bei zunehmender Kontrastmittelkonzentration eher erreicht wird.

Bei der Suche nach Kontrastmitteln hat man im Stand der Technik mit freien Ionen der Übergangselemente (Mn2+, Cu2+, Fe3+, Cr3+ ua.) untersucht, musste jedoch weitergehende Pläne bezüglich des Einsatzes solcher Ionen schnell wegen ihre zu großen Toxizität im Körper und ihrer schlechten Löslichkeit im Bereich des physiologischen Blut-pHs (7,35-7,45) wieder aufgeben.

Im Rahmen der NMR haben sich heute im medizinischen Bereich wegen ihrer guten Kontrastmittelfähigkeit hauptsächlich Gadolinium-haltige Kontrastmittel etabliert.

Da das Gadoliniumion in Form seines Chlorids, Sulphats oder Acetats jedoch toxisch wirkt und im retikuloendothelialen System (Monozyten-Makrophagensystem) sowie in Leber, Knochen und Milz angereichert wird, wird es stets in Form-eines Chelats appliziert, etwa als Gadolinium-Diäthylentraminpentaazetat (Gd-DTPA).

In seiner chemischen Struktur sitzt das Gadolinum 3+ Ion relativ dicht umgeben von anderen Molekülen inmitten des Chelats. Das Gadolinium-Ion hat insgesamt neun Koordinationsstellen.

Um kontrasterhöhend zu wirken, muss an das Gadolinium-Ion ein Wassermolekül 12 anbinden, damit der Abstand zwischen Wasserstoffkernen und dem Gd-Ion klein genug ist.

Nachteilhaft ist die starke Abschirmung des Gadolinium-Ions nach aussen durch den Chelatbildner. Dies behindert den freien, für die Kontrastverstärkung notwendigen Zugang von Wassermolekülen an das Gadolinium-Ion. Bricht man die Chelatbindung auf, so besteht die unmittelbare Gefahr der Freisetzung des toxischen Gadolinium-Ions, und eine Schädigung des Organismus wäre absehbar. Dem System "Gadolinium plus Komplexbildner" sind daher natürliche Grenzen bezüglich der NMR relevanten kontrastverstärkenden Wirkung gesetzt.

In dieser komplexgebundenen Form zirkuliert Gadolinium rein extrazellulär (Gefäße und Interstitium) und kann auch die (intakte) Blut-Hirn-Schranke nicht passieren. Ebenso wenig findet eine Penetration des Zellinneren statt. Zurückgeführt wird dieses Verhalten auf die ausgeprägte Hydrophilie der Verbindung.

Die Ausscheidung erfolgt durch glomeruläre Filtration über die Niere mit einer Halbwertszeit von etwa 90 min. Tubuläre Sezernierung oder Resorption wurde nicht beobachtet. Gd-Chelate können daher theoretisch als Kontrastmittel für NIRT-Urogramme verwendet werden. Die Dialysierbarkeit von Gd-DTPA ist im gleichen Maße gegeben, die Ausscheidung erfolgt in etwa derselben Zeit wie es bei Nierengesunden der Fall ist.

Die Bindung im Chelat gilt als ausreichend stabil, die Stabilitätskonstante wird mit 10²² angegeben. Eine Lebensdauer dieser Verbindung wird für fünf Jahre garantiert.

Gd-DTPA ist am Markt kommerziell beispielsweise unter der Produktbezeichnung "Magnevist^{™}" erhältlich. Eine Beschreibung dieses Kontrastmittels hinsichtlich seiner Wirkungsweise als Kontrastverstärker findet sich in "Felix Roland, Heshiki Atsuko et.al., (Herausgeber): 'Magnevist', Blackwell Science, 3. Auflage 1998, S. 1 bis 27." Insoweit kann zum allgemeinen Verständnis und zu weiteren technischen Einzelheiten darauf Bezug genommen werden.

Gd-DTPA in freier Form bindet Calcium und Magnesium, jeweils in ionisierter Form, was eine drastische Verschiebung im Elektrolythaushalt bedeutet, und etwa zu Herzrhytmusstörungen bis hin zum Herzstillstand führen kann.

In biochemischen Tests hat sich Gd-DTPA bisher als durchaus inert gezeigt, Verdrängungen aus dieser Verbindung durch andere Ionen sind noch nicht beobachtet worden. Metabolite dieser Verbindung sind ebenfalls noch nicht bekannt wie Formänderungen. Ein Restrisiko für den Patienten kann jedoch nicht ausgeschlossen werden.

Desweiteren ist Gd-DTPA in der Anwendung in der medizinischen Diagnostik aufgrund des teuren Konplexbildners und den hohen Ansprüchen an die Reinheit sehr teuer.

Außerordentlich wünschenswert in bestimmten Situationen der medizinischen Praxis ist es jedoch, den Kontrast im Rahmen der Bildgebung zu erhöhen, da damit die Messergebnisse als Basis für die medizinische Diagnostik klarer darstellbar und damit einfacher zu interpretieren sind.

Desweiteren ist es wünschenswert, neben der arteriellen/ venösen Applikation ein Kontrastmittel für die orale Applikation in der medizinischen NMR Diagnostik zur Verfügung zu haben. Dies ist derzeit mit Gd-DTPA nicht wirtschaftlich darstellbar, da die Menge des einzunehmenden Kontrastmittels bei oraler Applikation signifikant höher ist als bei arterieller/ venöser Applikation. Eine orale Applikation von Gd-DTPA ist daher zu teuer.

WO 02/20696 A1 (= EP 01 976 022.2) ist Stand der Technik im Sinne des Artikels 54 (3) EPÜ und betrifft die Synthese von Metallsalznanopartikeln mit einer organischen Flüssigkeit, wie einer phosphororganischen Verbindung, einem Monoalkylamin oder einem Dialkylamin, als Koordinationsmittel. Die Nanopartikel können mit Cer und Terbium als Dotandenpärchen dotiert sein. Ferner werden verschiedene dotierte Nanopartikel konkret genannt.

S. Morel et al. ("NMR relaxometric investigations of solid lipid nanoparticles (SLN) containing gadolinium (III) complexes" in European Journal of Pharmaceutics and Biopharmceutics, Bd. 45, Nr. 2, März 1998, Seiten 157-163) betrifft den Einsatz wasserlösliche Gadoliniumkomplexe bei NMR Untersuchungen.

US 5,496,536 betrifft ein Diagnoseverfahren auf NMR-Basis für Krankheiten, welche die Lymphknoten beeinträchtigen, bei dem eine diagnostisch wirksame Menge eines Kontrastmittels mit einer mittleren Teilchengrößen von etwa 5 bis etwa 900 nm verabreicht wird. Das Dokument zählt zahlreiche Verbindungsklassen auf, die sich als Kontrastmittel eignen, einschließlich von Oxiden, Phosphaten, Sulfiden oder Silikaten des Gadoliniums und paramagnetischen Gadoliniumteilchen.

US 6,048,515 betrifft Nanopartikel mit einem eisenhaltigen Kern. Eisenhaltige Nanopartikel und ihre Verwendung als Kontrastmittel in magnetischen Resonanzverfahren sind auch der Gegenstand von J. Bulte et al ("Magnetic Nanoparrticles as contrast agents for MR imaging", Scientific and Clinical Applications of Magentic carriers, 1997, Seiten 527-543).

H. Tokumitsu et al ("Gadolinium neutron-capture therapy using novel gadopentetic acid-chitosan complex nanoparticles: in vivo growth supression of experimental melanoma solid tumor" in Cancer Letters 150 (2000), 177 bis 182) bezieht sich auf die Gadoliniumneutroneneinfangtherapie, bei der in den Experimenten Magnevist® eingesetzt wurde. Auch X.Yu et al ("High resolution MRI characterization of human thrombus using a novel fibrin-targeted paramagnetic nanoparticle contrast agent", Magnetic Resonance in Medicine 44:867-872 (2000)) bezieht sich auf einen Gd-DPTA-Komplex.

I. Coroiu ("Relaxivities of different supermagnetic particles for application in NMR tomography" in Journal of Magnetism and Magnetic Materials 201 (1999) 449-452) untersucht die Relationszeiten verschiedener superparamagnetischer Teilchen hinsichtlich ihrer Anwendung in der NMR-Tomographie. Unter den hinsichtlich ihrer Größe näher beschriebenen Teilchen sind auch 5Fe₂O₃ x 3 Gd₂O₃-Teilchen, die nach einem Mikroemulsionsverfahren in einer Größe von 25-40 Å (2,5-4nm), in Dextran stabilisiert hergestellt wurden. Für die Dysprosium- und Gadoliniumphosphate wird ein Größenbereich von 40-300 Å (4-30 nm) angegeben.

D. Neogy et al ("Experimental and theoretical studies on the magnetic behaviour of Nd 3+ in NdPO4" in Journal of Magnetism and Magnetic Materials 173 (1997) 167-172) offenbarten Neodymphosphatteilchen mit einem Teilchendurchmesser im mm Bereich.

### ZUSAMMENFASSUNG DER ERFINDUNG

Die vorliegende Erfindung betrifft paramagnetische Nanopartikel mit einer Gitterstruktur aus Anionen- und Kationenbestandteilen, die eine Seltenerdverbindung ausgewählt unter Gadolinium, Praseodym, Erbium, Europium, Neodym, Terbium, Samarium und Dysprosium enthält und worin der Anionenbestandteil unter Boraten, Aluminaten, Gallaten, Silikaten, Germanaten, Phosphaten, Halophosphaten, Arsenaten, Vanadaten, Niobaten, Tantalaten, Sulfaten, Wolframaten, Molybdaten, Halogeniden und Nitriden ausgewählt wird und die Nanopartikel eine Größe von 1 bis 20nm mit einer Standardabweichung von weniger als 30% aufweisen, sowie zwei Verfahren zu ihrer Herstellung, wobei jeweils die in den Ansprüchen konkret genannten Verbindungen ausgenommen sind.

Die Erfindung betrifft ferner die Verwendung paramagnetischer Nanopartikel mit einer Gitterstruktur aus Anionen- und Kationenbestandteilen, die ein Seltenerdmetall enthält, worin der Anionenbestandteil unter Boraten, Aluminaten, Gallaten, Silikaten, Germanaten, Phosphaten, Halophosphaten, Arsenaten, Vanadaten, Niobaten, Tantalaten, Sulfaten, Wolframaten, Molybdaten, Halogeniden und Nitriden ausgewählt wird und die Nanopartikel eine Größe von 1 bis 20nm mit einer Standardabweichung von weniger als 30% aufweisen, als Kontrastmittel bei kernresonanzbasierten Untersuchungen, z.B. bei der zerstörungsfreien Werkstoffprüfung oder der medizinischen Diagnose.

### VORTEILE DER ERFINDUNG

Nanopartikel gemäß Anspruch 1, 2 oder 3 weisen gegenüber den bekannten Lösungsansätzen die Vorteile auf, dass sie eng grössenverteilt - mittlere Abweichungen um 1 nm mit geringer Größe, beispielsweise in einem Bereich zwischen 2 und 15 nm herstellbar sind, dass sie nicht agglomerieren und daher für vielerlei technische Anwendungen - nicht beschränkt auf NMR-spezifische Anwendungen - sehr gut geeignet sind.

Bezogen auf NMR-spezifische Anwendungen haben paramagnetische Nanopartikel - hergestellt nach der vorliegenden Erfindung oder nach anderen Herstellungsverfahren vom Stand der Technik den Vorteil, dass sie die medizinische MRT Diagnostik mit kontrastverbesserten Bildern versorgen können, dass sie die Kosten beträchtlich senken können und eine Gefährdung der Gesundheit des Menschen durch Toxizität des Kontrastmittels verringern. Gefahren, die sich durch freies DTPA ergeben können, sind bei der erfindungsgemäßen Verwendung von Nanopartikeln ohne DTPA-Komplex zwangsläufig ausgeschlossen. Auch liegt das den Nanoteilchen zugrunde liegende Seltenerd-Element nicht in freier Form vor, sondern eingebaut in ein Kristallgitter, so dass eine Wechselwirkung mit dem Körper des Patienten aufgrund der geringen Löslichkeit nicht möglich ist.

Zwei Syntheseverfahren - einerseits mit Wasser und andererseits mit organischem Lösungsmittel - werden für die Nanopartikel erfindungsgemäß vorgestellt. Sie führen beide zu Nanopartikeln aus einem engen Größenbereich, die nicht agglomerieren und in beliebigen Trägerfluiden homogen verteilbar sind - was für viele Anwendungen eine unverzichtbare Voraussetzung ist.

Dabei sind die Herstellungsschritte nicht spezifisch für NMR taugliche Nanopartikel mit kontrastverstärkender Wirkung. Es können ebenso andere Wirkungen physikalischer oder chemischer Art erzielt werden, etwa Fluoreszenz, bei entsprechender Dotierung.

Gemäß weiteren Nebenaspekten der vorliegenden Erfindung können mit den erfindungsgemäßen Nanopartikeln etliche weitere Anwendungsbereiche und Vorteile erzielt werden, die sämtlich auf charakteristischen Eigenschaften der erfindungsgemäßen Nanopartikel beruhen:
a. ihrer hervorragenden Kernresonanzfähigkeit bei signifikant weniger Materialeinsatz als im Stand der Technik, und
b. einer homogenen Verteilungsmöglichkeit der Nanopartikel in irgendeinem Stoff.

So können beliebige Flüssigkeiten, beispielsweise als Zwischenprodukt bei der Herstellung irgendeines daraus zu formenden Gegenstandes mit erfindungsgemäßen Nanopartikeln versetzt werden und später mit NMRbasierten Untersuchungsverfahren auf nahezu beliebig kleine Materialfehler wie etwa Lufteinschlüsse darin, etc., untersucht werden. Solche Flüssigkeiten werden hierin auch als Werkstoff-Flüssigkeiten bezeichnet.

Ein Hauptaspekt der vorliegenden Erfindung ist die Verwendung von paramagnetischen Nanopartikeln zum Ziel der Erhöhung des Kontrastes bzw. zur Veränderung der Relaxationszeit eines zu untersuchenden Materials oder Gewebes. Eine bevorzugte Verwendung zielt auf eine Verwendung als MRT-Kontrastmittel in der medizinischen Diagnostik ab.

In vorteilhafter Weise benötigt der Einsatz der erfindungsgemäßen Nanopartikel keine Komplexbildner, um die Toxizität des Kontrastmittels zu verhindern, da der kontrasterhöhende Stoff- beispielsweise Gadolinium fest in ein Kristallgitter, etwa wie GDP04 in ein Monazitgitter, eingebaut ist. Trotz des stabilen Einbaus des kontrasterhöhenden Stoffes in ein Gitter ist dennoch gewährleistet, dass wegen des hohen Anteils an Oberflächenatomen mehr freie Koordinationsstellen für Wasserstoffatome vorhanden sind als im Stand der Technik (1/9).

Beispielsweise sitzen bei GDPO4 Nanopartikeln eines Durchmessers von 5 Nanometern ca. 40% aller Atome an der Oberfläche. Wenn das gesamte Nanopartikel nun aus 10000 Atomen besteht, so ergeben sich ca. 4000 Oberflächenatome. Davon beträgt der Anteil von Gadoliniumatomen bei GdP04 genau 20%. So bieten etwa 800 Gd-Atome für Wasserstoffatome. Hin zu kleineren Nanopartikelgrößen verbessert sich das Ergebnis noch weiter.

Bei gleicher absoluter Menge an kemresonanzverstärkendem Stoff, etwa Gadolinium in Form von Gadoliniumphosphat, ergeben sich erfindungsgemäß wesentlich mehr reaktive Gadoliniumzentren an der Oberfläche der Nanopartikel als im Komplexbildner vom Stand der Technik. Daher kann bei Verwendung des erfindungsgemäßen, nanopartikelbasierten Kontrastmittels die Dosis erniedrigt oder bei gleicher Materialdosis die Meßzeit verringert werden.

Weiter besteht die Möglichkeit, auf andere, ggf. preisgünstigere Substanzen als Gadolinium mit an sich geringer Kontrastwirkung zurückzugreifen, wenn durch den Einbau in Nanopartikel der Effekt rekompensiert wird.

In den Unteransprüchen finden sich vorteilhafte Weiterbildungen und Verbesserungen des jeweiligen Gegenstandes der Erfindung.

Ein weiterer Aspekt der vorliegenden Erfindung ist dadurch gekennzeichnet, daß in dem erfindungsgemäßen Herstellungsverfahren Metallchloride zur Gewinnung des kationischen Bestandteils des Gitters, oder ein Phosphat zur Gewinnung seines anionischen Bestandteils verwendet werden, und ein Säurefänger, bevorzugt ein Amin, besonders bevorzugt Tioctylamin (C₂₄H₅₁N) zur Synthese hinzugefügt wird. Verwendet man Chloridsalze, liegt die Ausbeute des Materials, bezogen auf die Menge der eingesetzten Metallsalze bei ca. 80%, was ein Herstellungsverfahren im industriellen Maßstab ermöglicht. Damit läßt sich in vorteilhafter Weise ein Gitter mit einem Seltenerdkation und anionischem Phophat herstellen.

Setzt man als Lösungsmittel für die Reaktion einen Phosphorsäureester ein, läßt sich das Wachstum der Nanopartikel kontrollieren. Die Verwendung eines Phosphorsäureesters liefert eine hohe Ausbeute eng größenverteilter Nanopartikel. Der Phosphorsäureester kann dabei sowohl stöchiometrisch in einem Verhältnis Metallchlorid : Phosphorsäureester von 1:1 bis zu einem Verhältnis von 1:unendlich eingesetzt werden.

Es können jedoch auch die in der gleichzeitig anhängigen PCT-Anmeldung PCT/DE 00/03130 mit dem Titel 'Dotierte Nanopartikel', sowie deren Fortsetzungsanmeldung, beide vom gleichen Anmelder, offenbarten Substanzen als Lösungsmittel bei der Gewinnung der erfindungsgemäßen Nanopartikel verwendet werden.

Der aus dem Verfahren gewonnene Nanopartikelstoff kann nach Ausfällen und Trocknen, beispielsweise durch Heißluft als weich zerbröselbares, sehr feinkörniges Pulverkonzentrat vorliegen, das dann seinerseits in eine Vielzahl von Trägerstoffen, insbesondere Trägerflüssigkeiten oder Werkstoff-Flüssigkeiten eingebettet werden kann, je nachdem, wie es der jeweilige Anwendungsfall erfordert. Damit können die Nanopartikel neben der Verwendung als Kontrastmittel auch in beliebige andere, insbesondere durch Giessen und andere formende Prozesse hergestellte Gegenstände, auch Folien, etc., eingearbeitet werden.

Ist der Schmelzpunkt des Werkstoffes zu hoch, so daß durch die hohe Schmelztemperatur die vorteilhaften Eigenschaften der Nanopartikel verlorengehen, so können die Nanopartikel durch Einwalzen fest mit der Oberfläche verbunden werden.

Bei Werkstoffen mit niedrigerem Schmelzpunkt kann durch Verrühren der Trägerflüssigkeit mit dem Werkstoff eine homogene Mischung erzielt werden, die später unter anderem zur zerstörungsfreien Werkstoffprüfung ausgenutzt werden kann.

Die Synthese der erfindungsgemäßen Nanopartikel kann auf organischer Basis oder auf wässriger Basis erfolgen. Beide Syntheseverfahren sind in der gleichzeitig anhängigen, internationalen Anmeldung PCT/DE 00/03130 mit dem Titel 'Dotierte Nanopartikel', sowie deren Fortsetzungsanmeldung, beide vom gleichen Anmelder, für eine große Anzahl unterschiedlicher Nanopartikel, insbesondere mit verschiedenen Dotierungen offenbart.

Ebenso können die dort offenbarten Herstellungsverfahren zur Herstellung von nicht-dotierten Nanopartikeln herangezogen werden, wie es vom Fachmann leicht erkannt werden kann, da die Dotierung der Nanopartikel nicht wesentlich ist für deren größengezielte Synthese, sei es eine organische oder wässrige.

In besonders vorteilhafter Weise können die hierin offenbarten und / oder beanspruchten Substanzen auch mit der wässrigen Synthese hergestellt werden, wie sie in der gleichzeitig anhängigen, deutschen Patentanmeldung DE 100 58 544.2 mit dem Titel 'Phasentransfer von Nanopartikeln', vom gleichen Anmelder offenbart ist.

Anhand der verschieden variierbaren Ausgangsstoffe ergeben sich erfindungsgemäß eine große Auswahl an Stoffen, enthaltend Nanopartikel mit Seltenerdverbindungen und insbesondere paramagnetische Nanopartikel, die vorzugsweise aber nicht ausschliesslich herstellbar sind mit dem Verfahren, wie sie in den Ansprüchen definiert sind.

Je nach chemischen und physikalischen Eigenschaften lassen sich diese Stoffe dann einer wirtschaftlichen Verwendung zielgerichtet zuführen. NMR Untersuchungen im Rahmen einer NIRT und solche zur zerstörungsfreien Werkstoffprüfung sind dabei die wesentlichen, derzeit erkennbaren Anwendungen für die paramagnetischen Nanopartikel. Weitere Anwendungen sind die in der oben erwähnten, internationalen Anmeldung genannten, die im Zusammenhang mit optischen (UV, VIS oder NIR) Eigenschaften der Nanopartikel, insbesondere der Fluoreszenzeigenschaften stehen.

Da die Nanopartikel in einem Größenbereich von 1 bis 20nm, und bevorzugt 4 bis 5 nm mit einer Standardabweichung geringer als 30 %, bevorzugt geringer als 10% vorliegen, kann die Effizienz der jeweilig angestrebten Wirkung der Nanopartikel verstärkt werden, wie es oben bereits beschrieben wurde. Auch sehr feine und gleichmässige Verteilungen der Nanopartikel in anderen Trägerstoffen oder Werkstoffen sind damit möglich. Dadurch wird die jeweils angestrebte technische Wirkung wirtschaftlich effizient eingesetzt.

Wenn der Stoff, insbesondere als Kontrastmittel verwendet, eine Phosphatverbindung enthält, ergibt sich der Vorteil einer relativ einfachen Herstellung und geringer Toxizität.

Aufgrund der guten Wirkung als Kontrastverstärker eignen sich Kontrastmittel enthaltend Gadoliniumphosphatnanopartikel in besonderem Maße für die medizinische MRT-Anwendung. Aber auch Neodymphosphat- und Europiumphosphatnanopartikel sind geeignet.

Die erfindungsgemäßen Nanopartikel eignen sich ebenso als Antikörper-Markierung für in vitro durchgeführte NMR Verfahren. Ebenso können Krebs- oder Entzündungszellen im Rahmen der Histologie nach Verfahren des Standes der Technik markiert werden. Hierbei werden sehr dünne, dem Patienten entnommene Gewebeschichten behandelt. Im Stand der Technik gelang dies bisher nur mit fluoreszierenden Markierungsnanopartikeln. Erfindungsgemäß könnte das Antikörpernachweisverfahren analog mit NMR sensitiven Nanopartikeln modifiziert werden.

Weitere Anwendungen sind: Verwendung der erfindungsgemäßen Nanopartikel als MRT-Kontrastmittel zur in vivo Untersuchung zum Nachweis von Antikörpern durch Kopplung der Nanopartikel an diese. Ferner eignen sich die erfindungsgemäßen Nanopartikel als NMR und MRT Antikörpermarkierung zugleich.
Die erfindungsgemäßen nanopartikel eignen sich als NNlIt und MRT Antikörpermarkierung in vitro und als MRT Antiköropermarkierung in vivo. Durch die zusätzliche NMR Analysierbarkeit ergibt sich eine bessere Eigenschaft als Diagnostikum im Hinblick auf ein exakteres Meßergebnis, als mit Fluoreszenz erzielbar. Die gleichzeitige in vivo und in vitro Verwendung erspart dem Hersteller der Antikörper Kosten für die Entwicklung eines Antikörpers für den jeweils anderen Verwendungszweck.

Wenn die erfindungsgemäß hergestellten Nanopartikel in eine Trägerflüssigkeit eingebracht werden, können sie in einem vorgegebenen Anteil verdünnt und in einem anderen Medium wie etwa Gummi, Polymere, etc., verteilt werden.

Darauf basiert dann eine erfindungsgemäße Verwendung dieser Flüssigkeit zur Herstellung von form- und gießbaren Gegenständen, die später per NMR-Verfahren auf Störstellen im Materialinneren untersucht werden können, etwa hochbelastbare Fahrzeugreifen, oder Dichtungsmaterial, etc.

### ZEICHNUNG

Ausführungsbeispiele der Erfindung sind teilweise in der Zeichnung dargestellt und in der nachfolgenden Beschreibung näher erläutert.

Es zeigt:

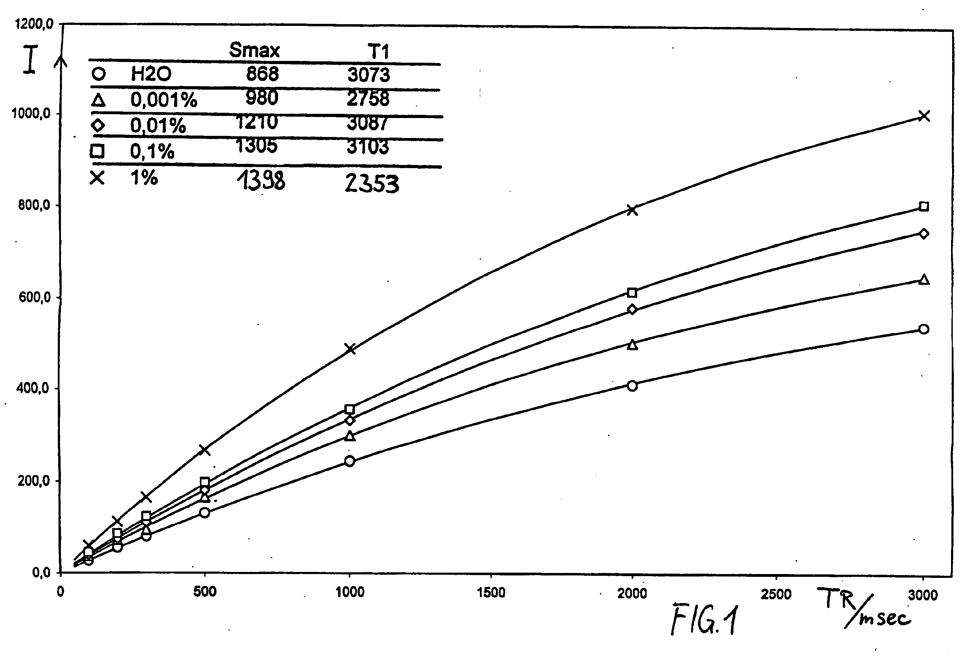

- Fig. 1: eine Meßkurvenschar: Signalstärke über verschiedenen T1-Zeiten mit verschiedenen Gewichtsprozent-Anteilen von Neodymphosphatnanopartikeln als kontrastverstärkende Substanz bei MRT Messungen.

### BESCHREIBUNG DER AUSFÜHRUNGSBEISPIELE

Im folgenden wird ein wesentlicher Aspekt der Erfindung näher erläutert, der zu den erfinderischen Vorteilen insbesondere in Bezug auf eine verbesserte MRT Diagnostik oder allgemein NMR Untersuchbarkeit beiträgt.

Nanopartikel befinden sich bezüglich ihrer Größe im Grenzbereich zwischen einzelnen Molekülen und makroskopischen Festkörpern mit typischerweise 10 bis 1000 Atomen. Nanopartikel können insbesondere in Lösungen hergestellt werden.

Fig. 1 zeigt für verschiedene, auf der X-Achse variierte T1-Zeiten (TR= Antwortzeiten in msec) die jeweiligen für eine MRT-Diagnose zugrundeliegenden Signalintensitäten in Wasser. Die Kreise zeigen die Signalintensität ohne Kontrastmittelzugabe, die Dreiecke stellen die Signalintensität bei Zugabe eines Kontrastmittels mit einem 0,001 Gewichtsprozentanteil von Neodymphosphatnanopartikeln dar, die erfindungsgemäß hergestellt wurden. Die Rautensymbole zeigen die Intensität bei einem Gewichtsprozentanteil von 0,01 %, die Vierecke zeigen die Signalintensität bei einem Gewichtsprozentanteil von 0,1 % und die Kreuze die Signalintensität bei einem Gewichtsprozentanteil von 1 %. Wie aus einem Vergleich über verschiedene T1-Zeiten hervorgeht, bewirkt die 1 %ige Zugabe über den gesamten T1-Hereich hinweg eine Steigerung, die zwischen 100 und 200 % liegt. Wie ebenfalls aus der Zeichnung hervorgeht, bewirkt schon eine geringfügige Zugabe von 0,001 Gewichtsprozent eine erhebliche Verstärkung der Signalintensität von jedenfalls mehr als 20 %.

Mit Gadoliniumphosphatnanopartikeln lassen sich noch höhere Werte der Signalverstärkung erreichen. Im folgenden werden eine Reihe von Herstellungsverfahren offenbart, die für beispielhaft ausgewählte Verbindungen stehen. Dazu sei angemerkt, daß der Schutzumfang der vorliegenden Erfindung nicht auf diejenigen Substanzen oder auf die erfindungsgemäße Verwendung derselben begrenzt ist, deren Herstellungsverfahren hiernach folgend explizit angegeben ist. Vielmehr kann ein erfindungsgemäßer Erfolg erreicht werden durch systematische Variation der Zusammensetzung von erfindungsgemäßen Nanopartikeln mit Metallionen, die paramagnetisch sind, und insbesondere mit Seltenerdelementen, sowie der folgenden Liste von 'Gegenionen', die zusammen eine Kristallstruktur ausbilden, die den oben genannten, vorteilhaften Gesichtspunkten genügt:

Als Gegenionen können Borate, Aluminate, Gallate, Silicate, Germanate, Phosphate, Halophosphate, Arsenate, Vanadate, Niobate, Tantalate, Sulfate, Wolframate, Molybdate, Halogenide und Nitride verwendet werden.

Es folgt die Offenbarung beispielhaft ausgewählter Herstellungsverfahren für verschiedene Nanopartikeltypen:

### 1. Synthese von GdPO4 Nanopartikeln

Vor der eigentlichen Synthese werden 1,176 g (12 mmol) H3PO4 mit 7,5 ml tetraglyme (Tetraethylenglykoldimethylether) versetzt und verschlossen 12 Stunden lang gerührt, bis eine klare Lösung entstanden ist.

Danach werden 3,71 g (10 mmol) GdCl3 • 6H2O in ca. 6 ml MeOH gelöst. Anschließend wird das gelöste Salz in einen 250 ml Kolben gegeben und mit 100 ml Trisethylhexylphosphat versetzt. Das MeOH wird dann vorsichtig im Vakuum bei RT abgezogen. Anschließend wird das Kristallwasser bei 30 ° C im Vakuum abdestilliert, bis die Lösung keine Blasen mehr bildet. Danach wird der Kolbenmit Stickstoff (N2) belüftet und 15,7 ml (36 mmol) Trioctylamin zu der Lösung gegeben. Anschließend wird das Phosphorsäure/Tetraglymegemisch vollständig zugegeben, die Apparatur verschlossen und unter Stickstoff ca. 40 h auf 473 Kelvin erhitzt.

### Aufarbeitung:

Die abgekühlte Lösung mit Methanol versetzen, zentrifugieren und dekantieren. Den Niederschlag vorsichtig mit analysereinem Methanol waschen, trocknen (keine hohen Temperaturen) und auswiegen.

### 2. Synthese von GdTaO₄ Kolloide:

Vorschrift zur Herstellung von K₈Ta₆O₁₉ · 16 H₂O (Mw = 1990.07 g/mol): Ofen auf 773 K vorheizen. 25 g KOH und 5 g Ta₂O₅ in einen Silbertiegel füllen und 30 min im Ofen zugedeckt (Ag-Blech) erhitzen (bis zum klaren Schmelzfluß !) . Währenddessen 500 ml dest. Wasser zum Sieden erhitzen. Den Tiegel aus dem Ofen nehmen, abkühlen lassen, und den Schmelzkuchen mehrmals mit wenig heißem Wasser (insgesamt etwa 50-100 ml, wenn es reicht) auslaugen. Die Lösung dabei in eine PE-Flasche (kein Glas !) füllen. Die Lösung durch ein Faltenfilter und Plastiktrichter in eine PE-Flasche filtrieren. Zum Ausfällen des Produktes die Lösung mit dem gleichen bis vierfachen Volumen an Ethanol (technisches funktioniert) versetzen. Die überstehende Lösung dekantieren, falls nötig nach Zentrifugation. Den Niederschlag noch zweimal in ca. 0.1 M KOH auflösen und mit Ehtanol ausfällen. Auf Filterpapier im Exsikkator (Kieselgel) trocknen und in eine Flasche füllen. (100% Ausbeute = 7.5 g nicht erreichbar wegen KTaO₃-Bildung).

### Vorschrift für GdTaO₄:

2.116 g (5 mMol) Gd(NO₃)₃ · 5 H2O in 20 ml Wasser lösen und zu 14 ml 1 M KOH in einem Teflon-Autoklavengefäß geben. 1.66 g K₈Ta₆O₁₉ · 16 H₂O (5 mMol Ta) und 1 ml 1 M KOH in 35 ml Wasser lösen und zur Lanthanid-Lösung geben. Die Lösung im Autoklaven (Teflongefäß) unter Rühren eine Stunde auf 543 K erhitzen. Den Niederschlag abfiltrieren und in 200 ml 0.5 HNO₃ (pH 0.3), die mit 6.87 g Dequest 2010-Lösung (60%ig) (20 mMol) versetzt ist, 60 min rühren. Danach mit mehr als 1 M KOH (bei 1 M ca. 80-200 ml !) auf einen pH-Wert von 12.5 bringen, über Nacht rühren und 10 min bei 4500 U/min zentrifugieren. Den Überstand vollständig abgießen und verwerfen.

Den Niederschlag mit 40 ml Wasser aufrühren und 2 min im Ultraschallbad dispergieren. Anschließend 15 min bei 4500 U/min zentrifugieren und dekantieren (Peptisierung ?). Den Überstand aufheben. Mit dem Niederschlag das Aufrühren und Abzentrifugieren noch dreimal wiederholen. Anschließend solange mit dest. Wasser waschen, bis Peptisation (= kleine Teilchen lösen sich wieder) einsetzt. Die kolloidale Lösung 60 min bei 12000 g zentrifugieren und den Niederschlag der Nanoteilchen durch Dekantieren vom Überstand trennen.

### 3. Synthese von GdV04-Kolloide:

### Vorschrift für GdVO₄

4.333 g (9.5 mMol) Gd(NO₃)₃ · 5 H₂O in 20 ml Wasser lösen und zu 15 ml 1 M NaOH in einem Teflon-Autoklavengefäß geben. 1.820 g Na₃VO₄ · 10 H₂O (5 mMol) in 35 ml Wasser lösen und zur Lanthanid-Lösung geben. Die Lösung im Autoklaven (Teflongefäß) unter Rühren eine Stunde auf 543 K erhitzen. Den Niederschlag abfiltrieren und in 100 ml 0.5 M HNO₃, die mit 6.87 g Dequest 2010-Lösung (60%ig) (Monsanto) (20 mMol) versetzt ist, 60 min rühren. Danach mit 1 M NaOH (ca. 40-100 ml !) auf pH 5 bringen und den Niederschlag 15 min bei 4500 U/min abzentrifugieren. Anschließend mit dest. Wasser waschen, bis Peptisation (= kleine Teilchen lösen sich wieder) einsetzt. Die kolloidale Lösung 60 min bei 12000 g zentrifugieren und den Niederschlag der Nanoteilchen durch Dekantieren vom Überstand trennen.

### 4. Synthese von Gd₃Ga₅O₁₂-Nanoteilchen:

3.89 g (10.4 mmol) Ga(NO₃)₃ · 6 H₂O, 2.68 g (5.9375 mmol) Gd(NO₃)₃ · 6 H₂O unter Rühren in 20 ml Wasser auflösen. Diese Lösung auf einen Satz in eine Lösung von 10 ml 25%iges Anmoniakwasser in 40 ml Wasser gießen (nicht umgekehrt!). Der pH-Wert muß größer als 10 sein, sonst noch konz. Ammoniak zugeben. Den Niederschlag abzentrifugieren, anschließend dekantieren. Den Niederschlag 5 Mal in 50-100 ml Wasser und anschließend 5 Mal in 50-100 ml Methanol aufrühren, waschen, zentrifugieren und dekantieren. Den dekantierten, aber noch methanolfeuchten Niederschlag zusammen mit 100 ml geschmolzenem 1,6-Hexandiol in eine Rückflußapparatur geben. Unter Vakuum auf 373 K erhitzen, bis alles Methanol und Wasser abdestilliert ist. Mit Inertgas (z.B. Stickstoff oder Argon) belüften und unter Inertgasstrom 16 Stunden unter Rückfluß kochen. Den Ansatz abkühlen lassen und in ein Glas für den Autoklaven überführen. Das Glas in den Autoklaven stellen und mit einer Glaskappe lose verschließen. Zum Wärmetransport 50 ml 1,6-Hexandiol in den Raum zwischen Autoklavenwand und Glas geben. Anschließend Autoklaven schließen, zweimal sorgfältig evakuieren und jeweils mit Stickstoff oder Argon (oder ein anderes Edelgas) befüllen. Schließlich den Autoklaven auf 573 K hochheizen und 4 Stunden bei dieser Temperatur halten. Den Autoklaven abkühlen lassen, dann den Inhalt des Glases in 100-250 ml Isopropanol auflösen. Den Niederschlag abzentrifugieren und mehrmals mit Isopropanol waschen. Anschließend mit dest. Wasser waschen, bis Peptisation (= kleine Teilchen lösen sich wieder) einsetzt. Die kolloidale Lösung 60 min bei 12000 g zentrifugieren und den Niederschlag der Gd₃Ga₅O₁₂:Tb Nanoteilchen durch Dekantieren vom Überstand trennen.

Die Reaktion funktioniert auch mit 1,4-Butandiol statt 1,6-Hexandiol, aber die Ausbeute an kleinen Teilchen wird schlechter.

### 5. Synthese von Y₃Al₅O₁₂: Nd Nanoteilchen:

4.26 g (20.8 mmol) Aluminiumisopropoxid, 4.15 g (11.875 mmol) Yttriumacetat · 4 H₂O und 215 mg (0.625 mmol) Neodym(III)acetat · 1,5 H₂O mit 100 ml 1,6-Hexandiol in ein Glas für den Autoklaven überführen. Das Glas in den Autoklaven stellen und mit einer Glaskappe lose verschließen. Zum Wärmetransport 50 ml 1,6-Hexandiol in den Raum zwischen Auotklavenwand und Glas geben. Anschließend Autoklaven schließen, zweimal sorgfältig evakuieren und jeweils mit Stickstoff oder Argon (oder ein anderes Edelgas) befüllen. Schließlich den Autoklaven auf 573 K hochheizen und 4 Stunden bei dieser Temperatur halten. Den Autoklaven abkühlen lassen, den Überdruck ablassen, dann erst öffnen. Den Inhalt des Glases in 100-250 ml Isopropanol auflösen. Den Niederschlag abzentrifugieren und mehrmals mit Isopropanol waschen. Anschließend mit dest. Wasser waschen, bis Peptisation (= kleine Teilchen lösen sich wieder) einsetzt. Die kolloidale Lösung 60 min bei 12000 g zentrifugieren und den Niederschlag der Y₃Al₅O₁₂:Nd-Nanoteilchen durch Dekantieren vom Überstand trennen.

Die Reaktion funktioniert auch mit 1,4-Butandiol statt 1,6-Hexandiol, aber die Ausbeute an kleinen Teilchen wird schlechter.

Ende der expliziten Herstellungsbeispiele.

Die erfindungsgemäßen Nanopartikel können nach Überführung in die Trägerflüssigkeit ihrer Anwendung zugeführt werden, etwa durch Schlucken oder intravenöse Applikation.

Weiter können die erfindungsgemäßen Nanopartikel auch in Produkte homogen verteilt eingebaut werden, die später sorgfältig auf Inhomogenitäten im Material untersucht werden müssen, um eine absolut zuverlässige Funktion des Produkts zu gewährleisten, z.B. für den Einsatz in der Raumfahrt, Flugzeugbau-, Formel 1- oder Flugzeug- Hochgeschwindigkeitsbereifung.

Ein solches Herstellungsverfahren für zerstörungsfrei prüfbare, und durch einen Form-, insbesondere einen Gußvorgang herstellbare Gegenstände, enthält dann im wesentlichen folgende Schritte:
a. Bereitstellen einer NMR-tauglichen Werkstoff-Flüssigkeit mit einem Bestandteil, der ein ungepaartes Elektron aufweist - siehe Beschreibung im Stand der Technik -Kapitel, oben - in einer vorgegebenen Menge, z.B. 500 Liter flüssiges Polymer,
b. Bereitstellen einer vorgegebenen Menge an Trägerflüssigkeit, z.B., 1 Liter zum Polymer passendes Lösungsmittel mit einer vorgegebenen Konzentration von Nanopartikeln, z.B. 5 Gewichtsprozent GdPO4 Nanopartikel vorherrschend in geringer Größe von etwa 5 Nanometern +/- 10 %,
c. Mischen von Trägerflüssigkeit und Werkstoff-Flüssigkeit, vorzugsweise bis eine homogene Verteilung der Nanopartikel in der Werkstoff-Flüssigkeit vorliegt, und
d. Formen/ Giessen des Gegenstandes.

Der hergestellte Gegenstand kann dann in vorteilhafter weise gründlich mit NMR-basierter Technik untersucht und 'durchleuchtet' werden. Inhomogenitäten im vollen Material fallen dabei auf. So können Lufteinschlüsse, feine Haar-Risse, etc. verläßlich gefunden werden, und das Produkt vorzugsweise nicht dem bestimmungsgemäßen Einsatz unterzogen werden. Dies erhöht die Sicherheit beim späteren Einsatz, weil nur Produkte sehr hoher Qualität eingesetzt werden.

Obwohl die vorliegende Erfindung anhand eines bevorzugten Ausführungsbeispiels vorstehend beschrieben wurde, ist sie darauf nicht beschränkt, sondern auf vielfältige Weise modifizierbar.

Wie es sich einem Durchschnittsfachmann auf dem einschlägigen Gebiet der Erfindung erschließen wird, können viele der oben genannten Herstellungsverfahren auf vielfältige Weise abgewandelt werden, um Nanopartikel, insbesondere paramagnetische Nanopartikel mit anderen Bestandteilen zu synthetisieren. Dabei werden bevorzugt die Ausgangsstoffe abgewandelt. So können etwa die kationischen Bestandteile durch Verwendung von Praseodym (PR), Neodym (Nd), Samarium (Sm), Europium (Eu), Terbium (Tb) oder Gadolinium (Gd) (wo vorher nicht vorhanden) variiert werden.

Analog können auch die anionischen Bestandteile gemäß der oben erwähnten Auswahlliste variiert werden, um verschiedene Stoffe zu erhalten. Bis auf wenige Ausnahmen können die Kationenbestandteile mit den Anionenbestandteilen frei kombiniert werden, wie es dem Durchschnittsfachmann aus der Makrowelt der Chemie bekannt ist.

Neben den Kostenvorteilen, die sich aus einer Materialeinsparung ergeben, die aufgrund der erhöhten Kontrastverstärkung eines erfindungsgemäßen Kontrastmittels erzielbar sind, ergibt auch die technisch wenig anspruchsvolle Herstellung von Nanopartikeln einen Kostenvorteil in der Produktion des erfinderischen Kontrastmittels, der von wirtschaftlicher Bedeutung ist.

Desweiteren von Bedeutung insbesondere für die medizinische Anwendung ist die Möglichkeit, verschiedene kontrastverstärkende Elemente zeitgleich einsetzen zu können, von denen man unterschiedliche Kontrast- und Anreicherungseigenschaften kennt oder zukünftig kennenlernen wird. Es lassen sich nunmehr - bedingt durch den stabilen Einbau der Ionen in ein Kristallgitter - verschiedene Elemente anwenden, die bislang aufgrund ihrer Toxizität für den Einsatz im Rahmen der med. Diagnostik nicht zur Verfügung gestanden haben.

Auch können je nach Wahl des kationischen Bestandteils zusätzlich zu den paramagnetischen Eigenschaften, die primär Gegenstand der vorliegenden Erfindung sind, auch Fluoreszenz-Leuchteigenschaften der synthetisierten Verbindung hinzukommen. Dies trifft vor allem zu für die Kationenbestandteile Eu, Tb, Sm, Nd, Erbium (Er) und Dysprosium (Dy).

## Patentansprüche

1. Paramagnetische Nanopartikel mit einer Gitterstruktur aus Anionen- und Kationenbestandteilen, die eine Seltenerdverbindung ausgewählt unter Gadolinium, Praseodym, Erbium, Europium, Neodym, Terbium, Samarium und Dysprosium enthält und worin der Anionenbestandteil unter Boraten, Aluminaten, Gallaten, Silikaten, Germanaten, Phosphaten, Halophosphaten, Arsenaten, Vanadaten, Niobaten, Tantalaten, Sulfaten, Wolframaten, Molybdaten, Halogeniden und Nitriden ausgewählt wird und die Nanopartikel eine Größe von 1 bis 20nm mit einer Standardabweichung von weniger als 30% aufweisen, und die folgenden Verbindungen ausgenommen werden: unter den Terbium-haltigen Verbindungen solche mit Cer und Terbium als Dotandenpärchen und ferner Sr₃Gd₂Si₆O₁₈:Pb, Mn; (Y,Gd)BO₃:Eu; GdVO₄:Eu; NaGdF₄:Yb,Er; Gd₃Ga₅O₁₂:Tb; Gd₃Ga₅O₁₂:Eu; GdTaO₄:Tb; LiI:Eu; BaFCl:Eu; BaFCl:Sm; BaFBr:Eu; BaFCl_{0,5}Br_{0,5}:Sm; BaY₂F₈:A(A= Pr, Er) ; BaMg₂Al₁₆O₂₇:Eu; BaMgAl₁₄O₂₃:Eu; BaMgAl₁₀O₁₇:Eu; (Ba, Mg)Al₂O₄:Eu; MgWO₄:Sm; CaF₂:Dy; CaWO₄:Sm; 2SrO.6(B₂O₃).SrF₂:Eu; 3Sr₃(PO₄)₂.CaCl₂:Eu; A₃(PO₄)₂.ACl₂:Eu (A=Sr, Ca, Ba); (Sr,Mg)₂P₂O₇:Eu; Sr₂P₂O₇:Eu; Sr₄Al₁₄O₂₅:Eu; YF₃:Yb,Er; YAl₃(BO₄)₃:Nd,Yb; (Y, Ga) BO₃:Eu; (Y,Gd)BO₃:Eu; Y₂Al₃Ga₂O₁₂:Tb; Y(P,V)O₄:Eu; YTaO₄:Nb; YAlO₃:A (A= Pr, Er); YOCl:Yb,Er; LuVO₄:Eu; GdVO₄:Eu; LaOBrTb; LaF₃:Nd,Ce; BaYb₂Fe:Eu; NaYF₄:Yb,Er; NaGdF₄:Yb,Er; NaLaF₄:Yb, Er; LaF₃:Yb,Er,Tm; BaYF₅:Yb,Er; GaN:A (A= Pr, Eu, Er, Tm); LiNbO₃:Nd,Yb; LiNbO₃:Er; LiLuF₄:A (A= Pr, Tm, Er) ; YVO₄:Eu; YVO₄:Sm; YVO₄:Dy; LaPO₄:Eu; Y₂SiO₅:Eu; Ca₃(PO₄)₂:Eu²⁺ ; Ca₃(PO₄)₂:Eu²⁺, Mn²⁺ ; Sr₂SiO₄:Eu²⁺; BaAl₂O₄:EU²⁺; Y₃Al₅O₁₂:Eu; Y₃Al₅O₁₂:Nd; Y₂(WO₄)₃:Eu; CaMoO₄:Eu; LaPO₄:Ce,Dy; Ca₃(PO₄)₂:Eu²⁺; Ca₃(PO₄)₂:Eu²⁺,Mn²⁺; BaAl₂O₄: Eu²⁺.

2. Paramagnetische Nanopartikel gemäß Anspruch 1, worin die Standardabweichung weniger als 10% beträgt.

3. Paramagnetische Nanopartikel gemäß Anspruch 1 oder 2, mit der Formel GdPO₄ oder GdVO₄.

4. Herstellungsverfahren für paramagnetische Nanopartikel mit einer Gitterstruktur aus Anionen- und Kationenbestandteilen, die eine Seltenerdverbihdung ausgewählt unter Gadolinium, Praseodym, Erbium, Europium, Neodym, Terbium, Samarium und Dysprosium enthält und worin der Anionenbestandteil unter Boraten, Aluminaten, Gallaten, Silikaten, Germanaten, Phosphaten, Halophosphaten, Arsenaten, Vanadaten, Niobaten, Tantalaten, Sulfaten, Wolframaten, Molybdaten, Halogeniden und Nitriden ausgewählt wird und die Nanopartikel eine Größe von 1 bis 20nm mit einer Standardabweichung von weniger als 30% aufweisen, mit der Ausnahme von GdVO₄:Eu; GdTaO₄:Tb; Y₂(WO₄)₃:Eu; CaMoO₄:Eu; und GdTaO₄:Tb;
wobei dieses Verfahren die folgenden Schritte umfasst:
a. Herstellen einer wässrigen Lösung des anionischen Bestandteils,
b. Herstellen einer wässrigen Lösung des kationischen Bestandteils,
c. Mischen beider Lösungen zu einer Gemischlösung,
d. Druckerhitzen der Lösung auf eine hohe Temperatur in einem Autoklaven,
e. Rühren der Gemischlösung während einer vorgegeben Zeitdauer bei der hohen Temperatur,
f. Gewinnen des Niederschlags von der Autoklavenwand,
g. Lösen des Niederschlag und Neutralisieren der Niederschlagslösung auf einen pH-Wert zwischen 4 und 6.
h. den Niederschlag von der Lösung trennen, den Niederschlag waschen bis Peptisation einsetzt,
i. Zentrifugieren des kolloidalen Lösung, und
k. Trennen der abgesetzten Nanopartikel vom Überstand.

5. Herstellungsverfahren gemäß Anspruch 4, worin die Temperatur in Schritt d oberhalb von 380K liegt.

6. Herstellungsverfahren gemäß Anspruch 4, worin in Schritt g der Niederschlag in HNO₃ gelöst wird.

7. Herstellungsverfahren gemäß Anspruch 4, worin in Schritt g der pH-Wert 5 beträgt.

8. Herstellungsverfahren für paramagnetische Nanopartikel mit einer Gitterstruktur aus Anionen- und Kationenbestandteilen, die eine Seltenerdverbindung ausgewählt unter Gadolinium, Praseodym, Erbium, Europium, Neodym, Terbium, Samarium und Dysprosium enthält und worin der Anionenbestandteil unter Boraten, Aluminaten, Gallaten, Silikaten, Germanaten, Phosphaten, Halophosphaten, Arsenaten, vanadaten, Niobaten, Tantalaten, Sulfaten, Wolframaten, Molybdaten, Halogeniden und Nitriden ausgewählt wird und die Nanopartikel eine Größe von 1 bis 20nm mit einer Standardabweichung von weniger als 30% aufweisen, wobei die folgenden Verbindungen ausgenommen werden:
unter den Terbium-haltigen Verbindungen solche mit Cer und Terbium als Dotandenpärchen und ferner Sr₃Gd₂Si₆O₁₈:Pb, Mn; (Y,Gd)BO₃:Eu; GdVO₄:Eu; NaGdF₄:Yb,Er; Gd₃Ga₅O₁₂:Tb; Gd₃Ga₅O₁₂:Eu; GdTaO₄:Tb; LiI:Eu; BaFCl:Eu; BaFCl:Sm; BaFBr:Eu; BaFCl_{0,5}Br_{0,5}:Sm; BaY₂F₈:A(A= Pr, Er); BaMg₂Al₁₆O₂₇:Eu; BaMgAl₁₄O₂₃:Eu; BaMgAl₁₀O₁₇:Eu; (Ba, Mg)Al₂O₄:Eu; MgWO₄:Sm; CaF₂:Dy; CaWO₄:Sm; 2SrO.6(B₂O₃).SrF₂:Eu; 3Sr₃(PO₄)₂.CaCl₂:Eu; A₃(PO₄)₂.ACl₂:Eu (A=Sr, Ca, Ba); (Sr,Mg)₂P₂O₇:Eu; Sr₂P₂O₇:Eu; Sr₄Al₁₄O₂₅:Eu; YF₃:Yb, Er; YAl₃(BO₄)₃:Nd,Yb; (Y,Ga)BO₃:Eu; (Y, Gd)BO₃:Eu; Y₂Al₃Ga₂O₁₂:Tb; Y(P,V)O₄:Eu; YTaO₄:Nb; YAlO₃:A (A= Pr, Er); YOCl:Yb,Er; LuVO₄:Eu; GdVO₄:Eu; LaOBrTb; LaF₃:Nd,Ce; BaYb₂F₈:Eu; NaYF₄:Yb, Er; NaGdF₄:Yb, Er; NaLaF₄:Yb, Er; LaF₃:Yb,Er,Tm; BaYF₅:Yb,Er; GaN:A (A= Pr, Eu, Er, Tm); LiNbO₃:Nd,Yb; LiNbO₃:Er; LiLuF₄:A (A= Pr, Tm, Er); YVO₄:Eu; YVO₄:Sm; YVO₄:Dy; LaPO₄:Eu; Y₂SiO₅:Eu; Ca₃(PO₄)₂:Eu²⁺; Ca₃(PO₄)₂:Eu²⁺, Mn²⁺ Sr₂SiO₄:Eu²⁺; BaAl₂O₄:Eu²; Y₃Al₅O₁₂:Eu; Y₃Al₅O₁₂:Nd; LaPO₄:Ce,Dy; Ca₃(PO₄)₂:Eu²⁺; Ca₃(PO₄)₂:Eu²⁺,Mn²⁺; BaAl₂O₄: Eu²⁺;
wobei das Verfahren als Koordinationsmittel für das Kation eine organische Flüssigkeit umfasst.

9. Herstellungsverfahren gemäß Anspruch 8, worin die organische Flüssigkeit unter einem Phosphorsäureester, Phosphorsäureamid, Phosphoramidoxid, Trialkylphosphin und Trialkylphosphinoxid ausgewählt wird.

10. Verfahren nach Anspruch 8 oder 9, enthaltend den Schritt, das Lösungsmittel in einem mindestens 6-fachen Molüberschuß zu verwenden.

11. Verfahren nach einem der Ansprüche 8 bis 10 zur Herstellung eines Phosphatnanopartikels, wobei ein Metallchlorid zur Gewinnung des kationischen Bestandteils des Gitters, Phosphorsäure (H₃PO₄) oder ein Phosphatsalz zur Gewinnung seines anionischen Bestandteils verwendet werden, und ein Säurefänger, bevorzugt ein Amin, besonders bevorzugt Trioctylamin (C₂₄H₅₁N) zur Synthesemischung hinzugefügt wird.

12. Verfahren nach Anspruch 9, worin das Phosphorsäureamid Hexamethylphosphorsäuretriamid ist.

13. Verfahren nach Anspruch 9, worin der Phosphorsäureester Trisethylhexylphosphat ist.

14. Verfahren nach Anspruch 9, wobei das Trialkylphosphin Trioctylphosphin (TOP) oder das Trialkylphosphinoxid Trioctylphosphinoxid (TOPO) ist.

15. Verfahren nach einem der vorstehenden Ansprüche, worin das Phosphoramid Tris-(dimethylamino)-phosphin ist.

16. Verwendung paramagnetischer Nanopartikel mit einer Gitterstruktur aus Anionen- und Kationenbestandteilen, die ein Seltenerdmetall enthält, worin der Anionenbestandteil unter Boraten, Aluminaten, Gallaten, Silikaten, Germanaten, Phosphaten, Halophosphaten, Arsenaten, Vanadaten, Niobaten, Tantalaten, Sulfaten, Wolframaten, Molybdaten; Halogeniden und Nitriden ausgewählt wird und die Nanopartikel eine Größe von 1 bis 20nm mit einer Standardabweichung von weniger als 30% aufweisen,
als Kontrastmittel bei kernresonanzbasierten Untersuchungen.

17. Verwendung paramagnetischer Nanopartikel mit einer Gitterstruktur aus Anionen- und Kationenbestandteilen, die ein Seltenerdmetall enthält, worin der Anionenbestandteil unter Boraten, Aluminaten, Gallaten, Silikaten, Germanaten, Phosphaten, Halophosphaten, Arsenaten, Vanadaten, Niobaten, Tantalaten, Sulfaten, Wolframaten, Molybdaten, Halogeniden und Nitriden ausgewählt wird und die Nanopartikel eine Größe von 1 bis 20nm mit einer Standardabweichung von weniger als 30% aufweisen,
zur zerstörungsfreien Werkstoffprüfung mit kernresonanzbasierten Untersuchungen.

18. Verwendung gemäß Anspruch 16 oder 17, worin das Seltenerdmetall Gadolinium ist.

19. Verwendung gemäß Anspruch 16 oder 17, worin das Seltenerdmetall unter Praseodym, Erbium, Europium, Neodym, Terbium, Samarium oder Dysprosium ausgewählt wird.

20. Verwendung gemäß einem der Ansprüche 16 bis 19, worin die Standardabweichung weniger als 10% ist.

21. Verwendung gemäß einem der Ansprüche 16 bis 20, worin die Nanopartikel die Formel GdPO₄ oder GdVO₄ aufweisen.

## Claims

1. Paramagnetic nanoparticles having a lattice structure of anion and cation constituents, which contains a rare earth compound selected from gadolinium, praseodymium, erbium, europium, neodymium, terbium, samarium and dysprosium, and wherein the anion constituent is selected from borates, aluminates, gallates, silicates, germanates, phosphates, halophosphates, arsenates, vanadates, niobates, tantalates, sulphates, tungstates, molybdates, halides and nitrides, and the nanoparticles have a size of 1 to 20 nm with a standard deviation of less than 30%, and the following compounds are exceptions: from the terbium-containing compounds, those having cerium and terbium as doping agent pairs and also Sr₃Gd₂Si₆O₁₈:Pb, Mn; (Y,Gd)BO₃:Eu; GdVO₄:Eu; NaGdF₄:Yb,Er; Gd₃Ga₅O₁₂:Tb; Gd₃Ga₅O₁₂:Eu; GdTaO₄:Tb; LiI:Eu; BaFCl:Eu; BaFCl:Sm; BaFBr:Eu; BaFCl_{0.5}Br_{0.5}:Sm; BaY₂F₈:A (A = Pr, Er); BaMg₂Al₁₆O₂₇:Eu; BaMgAl₁₄O₂₃:Eu; BaMgAl₁₀O₁₇:Eu; (Ba, Mg)Al₂O₄:Eu; MgWO₄:Sm; CaF₂:Dy; CaWO₄:Sm; 2SrO.6(B₂O₃).SrF₂:Eu; 3Sr₃(PO₄)₂.CaCl₂:Eu; A₃(PO₄)₂.ACl₂:Eu (A = Sr, Ca, Ba); (Sr, Mg)₃P₂O₇:Eu; Sr₂P₂O₇:Eu; Sr₄Al₁₄O₂₅:Eu; YF₃:Yb, Er; YAl₃(BO₄)₃:Nd,Yb; (Y, Ga)BO₃:Eu; (Y,Gd)BO₃:Eu; Y₂Al₃Ga₂O₁₂:Tb; Y(P,V)O₄:Eu; YTaO₄:Nb; YAlO₃:A (A = Pr, Er); YOCl:Yb,Er; LuVO₄:Eu; GdVO₄:Eu; LaOBrTb; LaF₃:Nd,Ce; BaYb₂F₈:Eu; NaYF₄:Yb,Er; NaGdF₄:Yb,Er; NaLaF₄:Yb,Er; LaF₃:Yb,Er,Tm; BaYF₅:Yb,Er; GaN:A (A = Pr, Eu, Er, Tm); LiNbO₃:Nd,Yb; LiNbO₃:Er; LiLuF₄:A (A = Pr, Tm, Er); YVO₄:Eu; YVO₄:Sm; YVO₄:Dy; LaPO₄:Eu; Y₂SiO₅:Eu; Ca₃(PO₄)₂:Eu²⁺; Ca₃(PO₄)₂:Eu²⁺, Mn²⁺; Sr₂SiO₄:Eu²⁺; BaAl₂O₄:Eu²⁺; Y₃Al₅O₁₂:Eu; Y₃Al₅O₁₂:Nd; Y₂(WO₄)₃:Eu; CaMoO₄:Eu; LaPO₄:Ce,Dy; Ca₃(PO₄)₂:Eu²⁺; Ca₃(PO₄)₂:Eu²⁺, Mn²⁺; BaAl₂O₄:Eu²⁺.

2. Paramagnetic nanoparticles according to claim 1, wherein the standard deviation is less than 10%.

3. Paramagnetic nanoparticles according to claim 1 or 2, having the formula GdPO₄ or GdVO₄.

4. Production process for paramagnetic nanoparticles having a lattice structure of anion and cation constituents, which contains a rare earth compound selected from gadolinium, praseodymium, erbium, europium, neodymium, terbium, samarium and dysprosium, and wherein the anion constituent is selected from borates, aluminates, gallates, silicates, germanates, phosphates, halophosphates, arsenates, vanadates, niobates, tantalates, sulphates, tungstates, molybdates, halides and nitrides, and the nanoparticles have a size of 1 to 20 nm with a standard deviation of less than 30%, with the exception of GdVO₄:Eu; GdTaO₄:Tb; Y₂(WO₄)₃:Eu; CaMoO₄:Eu; and GdTaO₄:Tb;
wherein this process comprises the following steps:
a. producing an aqueous solution of the anionic constituent,
b. producing an aqueous solution of the cationic constituent,
c. mixing both solutions to form a mixed solution,
d. pressure-heating the solution to a high temperature in an autoclave,
e. stirring the mixed solution for a preset period of time at the high temperature,
f. recovering the precipitate from the autoclave wall,
g. dissolving the precipitate and neutralising the precipitate solution to a pH value between 4 and 6,
h. separate the precipitate from the solution, wash the precipitate until peptisation starts,
i. centrifuging the colloidal solution, and
k. separating the deposited nanoparticles from the supernatant.

5. Production process according to claim 4, wherein the temperature in step d lies above 380 K.

6. Production process according to claim 4, wherein in step g, the precipitate is dissolved in HNO₃.

7. Production process according to claim 4, wherein in step g, the pH value is 5.

8. Production process for paramagnetic nanoparticles having a lattice structure of anion and cation constituents, which contains a rare earth compound selected from gadolinium, praseodymium, erbium, europium, neodymium, terbium, samarium and dysprosium, and wherein the anion constituent is selected from borates, aluminates, gallates, silicates, germanates, phosphates, halophosphates, arsenates, vanadates, niobates, tantalates, sulphates, tungstates, molybdates, halides and nitrides, and the nanoparticles have a size of 1 to 20 nm with a standard deviation of less than 30%, wherein the following compounds are exceptions:
from the terbium-containing compounds, those having cerium and terbium as doping agent pairs and also Sr₃Gd₂Si₆O₁₈:Pb, Mn; (Y,Gd)BO₃:Eu; GdVO₄:Eu; NaGdF₄:Yb,Er; Gd₃Ga₅O₁₂:Tb; Gd₃Ga₅O₁₂:Eu; GdTaO₄:Tb; LiI:Eu; BaFCl:Eu; BaFCl:Sm; BaFBr:Eu; BaFCl_{0.5}Br_{0.5}:Sm; BaY₂F₈:A (A = Pr, Er); BaMg₂Al₁₆O₂₇:Eu; BaMgAl₁₄O₂₃:Eu; BaMgAl₁₀O₁₇:Eu; (Ba, Mg)Al₂O₄:Eu; MgWO₄:Sm; CaF₂:Dy; CaWO₄:Sm; 2SrO.6(B₂O₃).SrF₂:Eu; 3Sr₃(PO₄)₂.CaCl₂:Eu; A₃(PO₄)₂.ACl₂:Eu (A = Sr, Ca, Ba); (Sr, Mg)₃P₂O₇:Eu; Sr₂P₂O₇:Eu; Sr₄Al₁₄O₂₅:Eu; YF₃:Yb, Er; YAl₃(BO₄)₃:Nd,Yb; (Y, Ga)BO₃:Eu; (Y,Gd)BO₃:Eu; Y₂Al₃Ga₂O₁₂:Tb; Y(P,V)O₄:Eu; YTaO₄:Nb; YAlO₃:A (A = Pr, Er); YOCI:Yb,Er; LuVO₄:Eu; GdVO₄:Eu; LaOBrTb; LaF₃:Nd,Ce; BaYb₂F₈:Eu; NaYF₄:Yb,Er; NaGdF₄:Yb,Er; NaLaF₄:Yb,Er; LaF₃:Yb,Er,Tm; BaYF₅:Yb,Er; GaN:A (A = Pr, Eu, Er, Tm); LiNbO₃:Nd,Yb; LiNbO₃:Er; LiLuF₄:A (A = Pr, Tm, Er); YVO₄:Eu; YVO₄:Sm; YVO₄:Dy; LaPO₄:Eu; Y₂SiO₅:Eu; Ca₃(PO₄)₂:Eu²⁺; Ca₃(PO₄)₂:Eu²⁺, Mn²⁺; Sr₂SiO₄:Eu²⁺; BaAl₂O₄:Eu²⁺; Y₃Al₅O₁₂:Eu; Y₃Al₅O₁₂:Nd; LaPO₄:Ce,Dy; Ca₃(PO₄)₂:Eu²⁺; Ca₃(PO₄)₂:Eu²⁺, Mn²⁺; BaAl₂O₄:Eu²⁺;
wherein the process comprises an organic liquid as coordination means for the cation.

9. Production process according to claim 8, wherein the organic liquid is selected from a phosphoric acid ester, phosphoric acid amide, amidophosphoric oxide, trialkylphosphine and trialkylphosphine oxide.

10. Process according to claim 8 or 9, containing the step of using the solvent in an at least 6 times molar excess.

11. Process according to one of claims 8 to 10 for producing a phosphate nanoparticle, wherein a metal chloride to recover the cationic constituent of the lattice, phosphoric acid (H₃PO₄) or a phosphate salt to recover its anionic constituent are used, and an acid acceptor, preferably an amine, particularly preferably trioctylamine (C₂₄H₅₁N) is added to the synthesis mixture.

12. Process according to claim 9, wherein the phosphoric acid amide is hexamethylphosphoric acid triamide.

13. Process according to claim 9, wherein the phosphoric acid ester is trisethylhexyl phosphate.

14. Process according to claim 9, wherein the trialkylphosphine is trioctylphosphine (TOP) or the trialkylphosphine oxide is trioctylphosphine oxide (TOPO).

15. Process according to one of the preceding claims, wherein the amidophosphorus is tris-(dimethylamino)-phosphine.

16. Use of paramagnetic nanoparticles having a lattice structure of anion and cation constituents, which contains a rare earth metal, wherein the anion constituent is selected from borates, aluminates, gallates, silicates, germanates, phosphates, halophosphates, arsenates, vanadates, niobates, tantalates, sulphates, tungstates, molybdates, halides and nitrides, and the nanoparticles have a size of 1 to 20 nm with a standard deviation of less than 30%, as contrast agents in nuclear resonance-based investigations.

17. Use of paramagnetic nanoparticles having a lattice structure of anion and cation constituents, which contains a rare earth metal, wherein the anion constituent is selected from borates, aluminates, gallates, silicates, germanates, phosphates, halophosphates, arsenates, vanadates, niobates, tantalates, sulphates, tungstates, molybdates, halides and nitrides, and the nanoparticles have a size of 1 to 20 nm with a standard deviation of less than 30%, for destruction-free material testing using nuclear resonance-based investigations.

18. Use according to claim 16 or 17, wherein the rare earth metal is gadolinium.

19. Use according to claim 16 or 17, wherein the rare earth metal is selected from praseodymium, erbium, europium, neodymium, terbium, samarium or dysprosium.

20. Use according to one of claims 16 to 19, wherein the standard deviation is less than 10%.

21. Use according to one of claims 16 to 20, wherein the nanoparticles have the formula GdPO₄ or GdVO₄.

## Revendications

1. Nanoparticules paramagnétiques avec une structure de réseau composée de constituants anioniques et cationiques, contenant une combinaison de terre rare sélectionnée parmi le gadolinium, praséodyme, erbium, europium, néodyme, terbium, samarium et dysprosium, et dans lesquelles le composé anionique est sélectionné parmi les borates, aluminates, gallates, silicates, germanates, phosphates, halophosphates, arsenates, vanadates, niobates, tantalates, sulfates, tungtanates, molybdates, halogénures et nitrures, et les particules nanométriques présentent une taille de 1 à 20 nm avec un écart type inférieur à 30 %, et les compositions suivantes étant exclues : parmi les compositions contenant du terbium, celles comprenant du Cer et du terbium en tant que appairages de dopants et, en outre, Sr₃Gd₂Si₆O₁₈:Pb, Mn ; (Y, Gd)BO₃:Eu ; GdVO₄:Eu ; NaGdF₄:Yb, Er ; Gd₃Ga₅O₁₂:Tb ; Gd₃Ga₅O₁₂:Eu ; GdTaO₄:Tb ; LiI:Eu ; BaFCl:Eu; BaFCl:Sm ; BaFBr:Eu ; BaFCl_{0,5}Br_{0,5}:Sm ; BaY₂F₈:A (A= Pr, Er) ; BaMg₂Al₁₆O₂₇:Eu ; BaMgAl₁₄O₂₃:Eu ; BaMgAl₁₀O₁₇:Eu ; (Ba, Mg)Al₂O₄;Eu ; MgWO₄:Sm ; CaF₂:Dy ; CaWO₄:Sm; 2SrO.6(B₂O₃).SrF₂:Eu ; 3Sr₃(PO₉)₂.CaCl₂:Eu ; A₃(PO₄)₂.ACl₂:Eu (A=Sr, Ca, Ba) ; (Sr, Mg)₂P₂O₇:Eu ; Sr₂P₂O₇:Eu ; Sr₄Al₁₄O₂₅:Eu ; YF₃:Yb, Er ; YAl₃(BO₄)₃:Nd,Yb ; (Y, Ga)BO₃:Eu ; (Y, Gd)BO₃:Eu ; Y₂Al₃Ga₂O₁₂:Tb ; Y(P,V)O₄:Eu ; YTaO₄:Nb ; YAlO₃:A (A= Pr, Er) ; YOCl:Yb, Er ; LuVO₄:Eu ; GdVO₄:Eu ; LaOBrTb ; LaF₃:Nd, Ce ; BaYb₂F₈:Eu ; NaYF₄:Yb, Er ; NaGdF₄:Yb, Er ; NaLaF₄:Yb,Er ; LaF₃:Yb, Er, Tm ; BaYb₅:Yb, Er ; GaN:A (A= Pr, Eu, Er, Tm) ; LiNbO₃:Nd, Yb ; LiNbO₃:Er ; LiLuF₄:A (A= Pr, Tm, Er) ; YVO₄:Eu ; YVO₄:Sm; YVO₄:Dy ; LaPO₄:Eu ; Y₂SiO₅:Eu ; Ca₃(PO₄)₂:Eu2⁺ ; Ca₃(PO₄)₂:Eu²⁺, Mn²⁺ ; Sr₂SiO₄:Eu²⁺ ; BaAl₂O₄:Eu²⁺ ; Y₃Al₅O₁₂:Eu ; Y₃Al₅O₁₂:Nd ; Y₂(WO₄)₃:Eu ; CaMoO₄:Eu ; LaPO₄:Ce,Dy ; Ca₃(PO₄)₂:Eu²⁺, Ca₃(PO₄)₂:Eu²⁺, Mn²⁺ ; BaAl₂O₄:Eu²⁺.

2. Nanoparticules paramagnétiques selon la revendication 1, dans lesquelles l'écart type est inférieur à 10 %.

3. Nanoparticules paramagnétiques selon la revendication 1 ou 2, de formule GdPO₄ ou GdVO₄.

4. Procédé de fabrication de nanoparticules paramagnétiques avec une structure de réseau composée de constituants anioniques et cationiques, contenant une combinaison de terre rare sélectionnée parmi le gadolinium, praséodyme, erbium, europium, néodyme, terbium, samarium et dysprosium, et dans lesquelles le composé anionique est sélectionné par les borates, aluminates, gallates, silicates, germanates, phosphates, halophosphates, arsenates, vanadates, niobates, tantalates, sulfates, tungtanates, molybdates, halogénures et nitrures, et les particules nanométriques présentent une taille de 1 à 20 nm avec un écart type inférieur à 30 %, à l'exception de GdVO₄:Eu ; GdTaO₄:Tb ; Y₂(WO₄)₃:Eu ; CaMoO₄:Eu ; et GdTaO₄:db ;
ce procédé comprenant les étapes suivantes :
a. préparation d'une solution aqueuse du composant anionique,
b. préparation d'une solution aqueuse du composant cationique,
c. mélange des deux solutions pour former une solution de mélange,
d. chauffage sous pression de la solution, à une température élevée dans un autoclave,
e. agitation de la solution mélangée pendant une durée prédéterminée à la température élevée,
f. récupération du dépôt sur la paroi d'autoclave,
g. dissoudre le dépôt et neutralisation de la solution de dépôt à une valeur de pH comprise entre 4 et 6.
h. séparation entre dépôt et solution, lavage du dépôt jusqu'à la peptisation,
i. centrifugation de la solution colloïdale, et
j. séparation des nanoparticules déposés du surnageant.

5. Procédé de préparation selon la revendication 4, dans lequel la température à l'étape d est supérieure à 380K.

6. Procédé de préparation selon la revendication 4, dans lequel à l'étape g, le dépôt est dissout dans du HNO₃.

7. Procédé de préparation selon la revendication 4, dans lequel à l'étape g, la valeur de pH est 5.

8. Procédé de préparation de nanoparticules paramagnétiques avec une structure de réseau composée de constituants anioniques et cationiques, contenant une combinaison de terre rare sélectionnée parmi le gadolinium, praséodyme, erbium, europium, néodyme, terbium, samarium et dysprosium, et dans lesquelles le composé anionique est sélectionné parmi les borates, aluminates, gallates, silicates, germanates, phosphates, halophosphates, arsenates, vanadates, niobates, tantalates, sulfates, tungtanates, molybdates, halogénures et nitrures, et les particules nanométriques présentent une taille de 1 à 20 nm avec un écart type inférieur à 30 %, et les compositions suivantes étant exclues : parmi les compositions contenant du terbium, celles comprenant du Cer et du terbium en tant que appairages de dopants et, en outre, Sr₃Gd₂Si₆O₁₈:Pb, Mn ; (Y, Gd)BO₃:Eu ; GdVO₄:Eu ; NaGdF₄:Yb, Er ; Gd₃Ga₅O₁₂:Tb ; Gd₃Ga₅O₁₂:Eu ; GdTaO₄:Tb ; LiI:Eu ; BaFCl;Eu; BaFCl:Sm ; BaFBr:Eu ; BaFCl_{0,5}Br_{0,5}:Sm ; BaY₂F₈:A(A= Pr, Er) ; BaMg₂Al₁₆O₂₇:Eu ; BaMgAl₁₄O₂₃:Eu ; BaMgAl₁₀O₁₇:Eu ; (Ba, Mg)Al₂O₄:Eu ; MgWO₄:Sm ; CaF₂:Dy ; CaWO₄:SM ; 2SrO.6(B₂O₃).SrF₂:Eu ; 3Sr₃(PO₄)₂.CaCl₂:Eu ; A₃(PO₄)₂.ACl₂:Eu (A=Sr, Ca, Ba) ; (Sr, Mg)₂P₂O₇:Eu ; Sr₂P₂O₇:Eu ; Sr₄Al₁₄O₂₅:Eu ; YF₃:Yb, Er ; YAl₃(BO₄)₃:Nd,Yb ; (Y, Ga)BO₃:Eu ; (Y, Gd)BO₃:Eu ; Y₂Al₃Ga₂O₁₂:Tb ; Y(P,V)O₄:Eu ; YTaO₄:Nb ; YAlO₃:A (A= Pr, Er) ; YOCl:Yb, Er ; LuVO₄:Eu ; GdVO₄:Eu ; LaOBrTb ; LaF₃:Nd, Ce ; BaYb₂F₈:Eu ; NaYF₄:Yb, Er ; NaGdF₄:Yb, Er ; NaLaF₄:Yb,Er ; LaF₃:Yb, Er, Tm ; BaYF₅:Yb, Er ; GaN:A (A= Pr, Eu, Er, Tm) ; LiNbO₃:Nd, Yb ; LiNbO₃:Er ; LiLuF₄:A (A= Pr, Tm, Er) ; YVO₄:Eu ; YVO₄:Sm ; YVO₄:Dy ; LaPO₄:Eu ; Y₂SiO₅:Eu ; Ca₃(PO₄)₂:Eu²⁺ ; Ca₃(PO₄)₂:Eu²⁺, Mn²⁺ ; Sr₂SiO₄:Eu²⁺ ; BaAl₂O₄:Eu²⁺ ; Y₃Al₅O₁₂:Eu ; Y₃Al₅O₁₂:Nd ; LaPO₄:Ce,Dy ; Ca₃(PO₄)₂:Eu²⁺, Ca₃(PO₄)₂:Eu²⁺, Mn²⁺ ; BaAl₂O₄:Eu²⁺.
le procédé comprenant un liquide organique, en tant qu'agent de coordination pour le cation.

9. Procédé de préparation selon la revendication 8, le liquide organique étant sélectionné parmi un ester d'acide phosphorique, un amide d'acide phosphorique, un dioxyde de phosphoreamide, une trialkylphosphine et un trialkylphosphinoxyde.

10. Procédé selon la revendication 8 ou 9, contenant l'étape d'utilisation du solvant en un excès molaire au moins sextuple.

11. Procédé selon l'une des revendications 8 à 10 pour la préparation d'une nanoparticule à base de phosphate, un chlorure métallique étant utilisé pour la récupération du composant cationique du réseau, de l'acide phosphorique (H₃PO₄) ou un sel de phosphate étant utilisé pour récupération de son composant anionique, et un capteur d'acidité, de préférence un amine, de façon particulièrement préférée du trioctylamine (C₂₉H₅₁N), étant ajouté au mélange de synthèse.

12. Procédé selon la revendication 9, l'amide d'acide phosphorique étant un triamide hexaméthyl acide phosphorique.

13. Procédé selon la revendication 9, dans lequel l'ester d'acide phosphorique est du triséthylhéxylphosphate.

14. Procédé selon la revendication 9, le trialkylphosphine étant du trioctylphosphine (TOP), ou le trialkylphosphine oxyde étant du trioctylphosphine oxyde (TOPO).

15. Procédé selon l'une des revendications précédentes, dans lequel le phosphoreamide est une tris-(diméthylamino)-phosphine.

16. Utilisation de nanoparticules paramagnétiques avec une structure de réseau composée de constituants anioniques et cationiques, contenant un métal des terres rares, dans lequel le composant anionique est sélectionné parmi les borates, aluminates, gallates, silicates, germanates, phosphates, halophosphates, arsénates, vanadates, niobates, tantalates, sulfates, tungtanates, molybdates, halogénures et nitrures, et les nanoparticules présentent une taille de 1 à 20 nm avec un écart type inférieur à 30 %,
en tant qu'agents de contraste pour des examens basés sur la résonance nucléaire.

17. Utilisation de nanoparticules paramagnétiques avec une structure de réseau composée de constituants anioniques et cationiques, contenant un métal des terres rares, le composé anionique étant sélectionné parmi les borates, aluminates, gallates, silicates, germanates, phosphates, halophosphates, arsenates, vanadates, niobates, tantalates, sulfates, tungtanates, molybdates, halogénures et nitrures, et les particules nanométriques présentent une taille de 1 à 20 nm avec un écart type inférieur à 30 %,
pour l'examen non destructif des matériaux avec des examens à base de résonance nucléaire.

18. Utilisation selon la revendication 16 ou 17, dans laquelle le métal des terres rares est du gadolinium.

19. Utilisation selon la revendication 16 ou 17, dans laquelle le métal des terres rares est sélectionné parmi le praséodyme, l'erbium, l'europium, le néodyme, terbium, samarium ou dysprosium.

20. Utilisation selon l'une des revendications 16 à 19, dans laquelle l'écart type est inférieur à 10 %.

21. Utilisation selon l'une des revendications 16 à 20, dans laquelle les nanoparticules présentent la formule GdPO₄ ou GdVO₄.
